# EUROPÄISCHE PATENTSCHRIFT

(11) **EP 2 417 089 B1**
(45) Veröffentlichungstag und Bekanntmachung des Hinweises auf die Patenterteilung: **05.06.2013**
(21) Anmeldenummer: 10711899.4
(22) Anmeldetag: 31.03.2010
(51) Int. Cl.: C07C 29/149, C07C 31/125, C07C 29/145, C07C 29/80, C07C 35/14, C07C 51/31, C07C 59/185, C07C 59/01, C07C 55/14, C07C 67/08, C07C 69/02, C07D 313/04, C07D 307/33

(54) **VERFAHREN ZUR HERSTELLUNG VON 1,6-HEXANDIOL UND CAPROLACTON**
METHOD FOR PRODUCING 1,6-HEXANEDIOL AND CAPROLACTONE
PROCÉDÉ DE PRODUCTION DE 1,6-HEXANEDIOL ET DE CAPROLACTONE

(30) Priorität: 07.04.2009 EP 09157503
(43) Veröffentlichungstag der Anmeldung: 15.02.2012
(73) Patentinhaber: BASF SE, 67056 Ludwigshafen (DE)
(72) Erfinder: PINKOS, Rolf, 67098 Bad Dürkheim (DE); BREUNINGER, Daniel, 67240 Bobenheim-Roxheim (DE); Dr. TEBBEN, Gerd-Dieter, 68239 Mannheim (DE)
(86) Internationale Anmeldenummer: PCT/EP2010/054285
(87) Internationale Veröffentlichungsnummer: WO 2010/115798

(56) Entgegenhaltungen:
- WO-A1-2006/005504
- WO-A1-2008/152001
- DE-A1-102004 054 047
- US-A- 3 933 930

## Beschreibung

Die Erfindung betrifft ein Verfahren zur Herstellung von 1,6-Hexandiol und Caprolacton, bevorzugt mit mindestens 99,5 %iger Reinheit, die insbesondere von 1,4-Cyclohexandiolen praktisch frei sind, aus einem Carbonsäuregemisch, das als Nebenprodukt der katalytischen Oxidation von Cyclohexan zu Cyclohexanon/Cyclohexanol mit Sauerstoff oder Sauerstoff enthaltenden Gasen und durch Wasserextraktion des Reaktionsgemisches erhalten wird, durch Hydrierung des Carbonsäuregemisches, Veresterung und Hydrierung eines Teilstroms zu 1,6-Hexandiol und Cyclisierung von 6-Hydroxycapronsäureester zu ε-Caprolacton, wobei die 1,4-Cyclohexandiole entweder bei der Fraktionierung des Veresterungsgemisches oder zuletzt vom Caprolacton abgetrennt werden.

1,6-Hexandiol stellt einen gesuchten Monomerbaustein dar, der überwiegend auf dem Polyester- und Polyurethansektor eingesetzt wird. ε-Caprolacton bzw. die daraus durch Polyaddition hergestellten Polycaprolactone dienen zur Herstellung von Polyurethanen.

Die wässrigen Lösungen von Carbonsäuren, die bei der katalytischen Oxidation von Cyclohexan zu Cyclohexanol und Cyclohexanon (vgl. Ullmann's Encyclopedia of Industrial Chemistry, 5. Ed., 1987, Vol. A8, S. 49) als Nebenprodukte entstehen, im folgenden Dicarbonsäurelösung (DCL) genannt, enthalten (berechnet wasserfrei in Gew.-%) im Allgemeinen zwischen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen 2 und 10 % Ameisensäure, zwischen 0,5 und 5 % 4-Hydroxycyclohexanon, zwischen 0,5 und 10 % 6-Oxocapronsäure sowie eine Vielzahl weiterer Mono- und Dicarbonsäuren, Ester, Oxo- und Oxa-Verbindungen, deren Einzeigehalte im Allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und 2- bzw. 3-Hydroxyadipinsäure genannt.

WO2008/152001 beschreibt ein Verfahren zur katalytischen Cyclisierung von Adipinsäure enthaltender 6-Hydroxycapronsäure zum ε-Caprolacton in der Gasphase.

DE 10 2004 054047 beschreibt ein Verfahren zur Herstellung von 1,6-Hexandiol aus Adipinsäure und 6-Hydroxycapronsäure enthaltenden Carbonsäuregemischen, die mit 1,4-Cyclohexandiol verunreinigt sind. Bei diesem Verfahren wird das Carbonsäuregemisch zunächst verestert, anschließend destillativ von 1,4-Cyclohexandiol befreit und schließlich zum Diol hydriert. WO 2006/005504 beschreibt ein ähnliches Verfahren, bei dem zudem die Verunreinigungen 1,4-Cyclohexandion und 4-Hydroxycyclohexan-1-on durch selektive Hydrierung nach dem Veresterungsschritt und vor Abtrennung des 1,4-Cyclohexandiol entfernt werden, wodurch besonders reines 1,6-Hexandiol erhältlich ist.

Aus DE 2 321 101 und DE 1 235 879 ist bekannt, diese wässrigen Dicarbonsäurelösungen bei Temperaturen von 120 bis 300°C und Drücken von 50 bis 700 bar in Gegenwart überwiegend Kobalt enthaltender Katalysatoren zu 1,6-Hexandiol als Hauptprodukt zu hydrieren. Die Hydrierausträge werden bevorzugt destillativ aufgearbeitet. Dabei gelingt es auch mit extrem hohem Destillationsaufwand nicht oder nur unvollständig, die bei der Hydrierung nicht veränderten 1,4-Cyclohexandiole von 1,6-Hexandiol zu trennen, so dass sich die 1,4-Cyclohexandiole, die anfänglich bereits in der DCL enthalten waren, mit einem Gehalt von im Allgemeinen 2 bis 5 Gew.-% im 1,6-Hexandiol wiederfinden.

Um diesem Problem zu begegnen, sind einige Lösungsansätze bekannt:
In US 3 933 930 wird die Umsetzung von 1,4-Cyclohexandiol in wässrigen Lösungen von Adipinsäure und 6-Hydroxycapronsäure zu Cyclohexanol, Cyclohexan und/oder Cyclohexen beschrieben, indem das Gemisch katalytisch vorhydriert wird. Dieses Verfahren bedarf des Einsatzes zweier verschiedener Hydrierkatalysatoren, eines für die Vorhydrierung, eines für die eigentliche Carbonsäurehydrierung und ist daher aufwändig.

Nach DE-OS 2 060 548 wird sehr reines 1,6-Hexandiol durch Kristallisation gewonnen. Auch dieses Verfahren ist sehr aufwändig und außerdem mit erheblichen Ausbeuteverlusten verbunden.

Eine weitere Möglichkeit zur Gewinnung von hochreinem 1,6-Hexandiol besteht darin, anstelle von DCL reine Adipinsäure oder reine Adipinsäureester zu hydrieren, wie es von K. Weissermel, H.J. Arpe in Industrielle Organische Chemie, VCH-Verlagsgemeinschaft Weinheim, 4. Auflage, Seite 263, 1994 beschrieben ist. Reine Adipinsäure ist jedoch im Vergleich zu DCL sehr teuer. Ferner ist das Carbonsäuregemisch, das bei der Cyclohexanoxidation anfällt, ein Abfallprodukt, das auch aus Umweltschutzgesichtspunkten einer stofflichen Verwertung zugeführt werden sollte. Aus Adipinsäure lässt sich auch kein Caprolacton auf einfache Weise gewinnen.

Caprolacton wird großtechnisch überwiegend auf Basis Cyclohexanon durch Baeyer-Villinger-Oxidation hergestellt. Dabei werden explosive Perverbindungen entweder eingesetzt oder im Verfahren durchlaufen.

Auch die Herstellung von Caprolacton aus DCL ist z.B. aus DE 1 618 143 bereits beschrieben worden. Dabei wird entwässerte DCL mit Phosphorsäure thermisch umgesetzt und ein Gemisch aus Dicarbonsäuren, Caprolacton sowie eine Vielzahl anderer Komponenten fraktioniert. Der Sumpf fällt dabei z.T. fest und schwerlöslich an. Das Caprolacton hat aber auch nach weiterer destillativer Aufarbeitung nur eine 98 %ige Reinheit.

Ferner ist in DE-A 2 013 525 wie auch in EP-A 349861 beschrieben 6-Hydroxycapronsäure oder deren Ester zu Caprolacton umzusetzen.

In DE-A 196 07 954 ist bereits ein Verfahren beschrieben, das den Zugang von 1,6-Hexandiol und Caprolacton aus o.g. wässrigen Carbonsäuregemischen beschreibt. Dieses an sich elegante Verfahren hat aber noch gewisse Nachteile. So werden nicht alle in der DCL vorhandenen linearen C6-Komponenten zur Herstellung von 1,6-Hexandiol bzw. Caprolacton genutzt. Beispielsweise geht die vorhandene 6-Oxocapronsäure im Prozess verloren und vermindert auch noch durch Hochsiederbildung die Destillationsausbeuten von zur Herstellung von 1,6-Hexandiol und gegebenenfalls Caprolacton notwendigen intermediären Estern. Ferner ist das 1,6-Hexandiol nicht völlig frei von unerwünschten 1,4-Cyclohexandiolen, da diese im Prozess zwar als solche effizient abgetrennt werden, jedoch als 4-Hydroxycyclohexanon in die Hydrierung gelangen und dort wiederum 1,4-Cyclohexandiole ergeben, die nur schlecht von 1,6-Hexandiol abgetrennt werden können. Außerdem sind Folgeprodukte der 6-Oxocapronsäure im 1,6-Hexandiol nachweisbar, wie z.B. 6,6-Dimethoxyhexan-1-ol und 6-Methoxyhexan-1-ol. Diese Monoalkohole sind bei Polymer-Anwendungen von Diolen im Allgemeinen sehr störend, da sie beim Kettenaufbau ein Ende blockieren. Ferner ist von Nachteil, dass die in der DCL vorhandene Ameisensäure bei der Wasserabtrennung vor der Veresterungsstufe Korrosionsprobleme verursacht, so dass hochwertige, teure Werkstoffe verwendet werden müssen.

Es bestand daher die Aufgabe, ein Verfahren zur Herstellung von 1,6-Hexandiol und Caprolacton bereit zu stellen, das es ermöglicht, auch von hoch komplexen Dicarbonsäurelösungen ausgehend, die darin enthaltenen linearen C6-Carbonsäuren möglichst vollständig zur Herstellung von sehr reinem 1,6-Hexandiol und sehr reinem Caprolacton umzusetzen und somit die gleiche bzw. höhere Reinheit der Produkte wie sie von dem Herstellungsverfahren ausgehend von reiner Adipinsäure bekannt sind, zu erreichen, ohne zusätzliche und kostenintensive Reinigungsschritte und/oder Materialen zu benötigen.

Diese Aufgabe wird gelöst durch eine Verfahren zur Herstellung von 1,6-Hexandiol und ε-Caprolacton aus einem Adipinsäure, 6-Hydroxycapronsäure, 6-Oxocapronsäure, 4-Hydroxycyclohexanon, Ameisensäure und bezogen auf die Summe an Adipinsäure und Hydroxycapronsäure zwischen 0,5 und 5 Gew.- % 1,4-Cyclohexandiole enthaltenden Carbonsäuregemisch, das als Nebenprodukt der katalytischen Oxidation von Cyclohexan zu Cyclohexanon/Cyclohexanol mit Sauerstoff oder Sauerstoff enthaltenden Gasen durch Wasserextraktion des Reaktionsgemisches erhalten wird, durch Veresterung und Hydrierung eines Teilstroms zu 1,6-Hexandiol und Cyclisierung von 6-Hydroxycapronsäureester zu ε-Caprolacton, dadurch gekennzeichnet, dass man
a) nur die im wässrige Carbonsäuregemisch enthaltenen Aldehyde und Ketone bei einer Hydrierungstemperatur von 50 bis 200 °C und einem Reaktionsdruck von 5 bis 35 bar absolut katalytisch zu den entsprechenden Alkoholen hydriert und gegebenenfalls enthaltene C-C-Doppelbindungen zu den entsprechenden gesättigten Verbindungen hydriert und mehr als 50 Gew.-% der im Gemisch enthaltenen Ameisensäure abbaut
b) die in dem wässrigen Reaktionsgemisch enthaltenen Mono- und Dicarbonsäuren nach Entwässerung mit einem niedermolekularen Alkohol zu den entsprechenden Carbonsäureestern umsetzt,
c) das erhaltene Veresterungsgemisch in einer ersten Destillationsstufe von überschüssigem Alkohol und Leichtsiedern befreit,
d) aus dem Sumpfprodukt in einer zweiten Destillationsstufe eine Auftrennung in eine von 1,4-Cyclohexandiolen abgereicherte Esterfraktion und eine 1,4-Cyclohexandiole enthaltende Fraktion durchführt,
e) von der Esterfraktion in einer dritten Destillationsstufe zumindest teilweise einen überwiegend 6-Hydroxycapronsäureester enthaltenden Strom abtrennt,
f) die Esterfraktion aus (e), aus der zumindest teilweise 6-Hydroxycapronsäureester entfernt wurde, katalytisch hydriert und durch Destillation des Hydrierproduktes in an sich bekannter Weise 1,6-Hexandiol gewinnt und
g) den überwiegend 6-Hydroxycapronsäureester enthaltenden Strom auf Temperaturen über 200°C bei vermindertem Druck erhitzt, wodurch 6-Hydroxycapronsäureester zu ε-Caprolacton cyclisiert wird und aus dem Cyclisierungsprodukt durch Destillation reines ε-Caprolacton gewinnt.

Die Hydrierung einer DCL ist sehr komplex, da viele Verbindungen enthalten sind, die die eigentliche Hydrierung stören können bzw. ebenfalls mithydriert werden, was die folgende Aufarbeitung erschweren kann. Es war nicht trivial und überraschend, dass die Hydrierung der Aldehyde in Schritt a) so selektiv erfolgte, dass die in der DCL enthaltenen C6-Hydroxycarbonsäuren nicht bereits in diesem Schritt zu 1,6-Hexandiol umgesetzt wurden. Wäre dies der Fall, würde das entstehende 1,6-Hexandiol anschließend in Schritt d) des erfindungsgemäßen Verfahrens zusammen mit den 1,4-Cyclohexandiolen abgetrennt werden und somit die Ausbeute an 1,6-Hexandiol verringern. Des Weiteren war es überraschend, dass der eingesetzte Katalysator trotz des korrosiven Mediums eine hohe Lebensdauer aufweist, die Bildung von Hochsiedern im Prozess so gesenkt werden konnte, dass die Ausbeute an 1,6-Hexandiol und Caprolacton deutlich verbessert wurde und dass die Reinheit von 1,6-Hexandiol entscheidend verbessert werden konnte. Des Weiteren war nicht vorherzusehen, dass Ameisensäure zumindest zu 50 % abgebaut wurde und damit nachfolgende Stufen weniger durch Korrosion betroffen sind.

Die Veresterung kann ohne Zusatz von Katalysatoren bevorzugt unter Einwirkung von Katalysatoren durchgeführt werden. Als niedermolekulare Alkohole kommen in der Regel solche mit 1 bis 10 C-Atomen, insbesondere Alkanole mit 1 bis 8 C-Atomen in Betracht. Auch Diole wie Butandiol oder Pentandiol kommen prinzipiell in Betracht. Soll Caprolacton gewonnen werden, kommen auch Alkohole, die höher als Caprolacton sieden, wie z.B. 1,6-Hexandiol, Octadecanol oder Trimethylolpropan in Betracht.

Die technisch bevorzugten für die Veresterung zu verwendenden Alkohole sind n- oder i-Butanol und insbesondere Methanol.

Im Falle der Veresterung mit Methanol geht man so vor, dass man in der Destillationsstufe (d) eine von 1,4-Cyclohexandiolen befreite Carbonsäuremethylesterfraktion am Kopf der Kolonne und eine die Hochsieder und die 1,4-Cyclohexandiole enthaltende Sumpffraktion gewinnt und die Carbonsäuremethylesterfraktion in der Hydrierstufe (f) katalytisch hydriert.

Im erfindungsgemäßen Verfahren bedeuten Begriffe wie über Kopf bzw. über Sumpf, jeweils den Abzug oberhalb bzw. unterhalb des Zulaufs einer Destillationseinheit wie einer Kolonne.

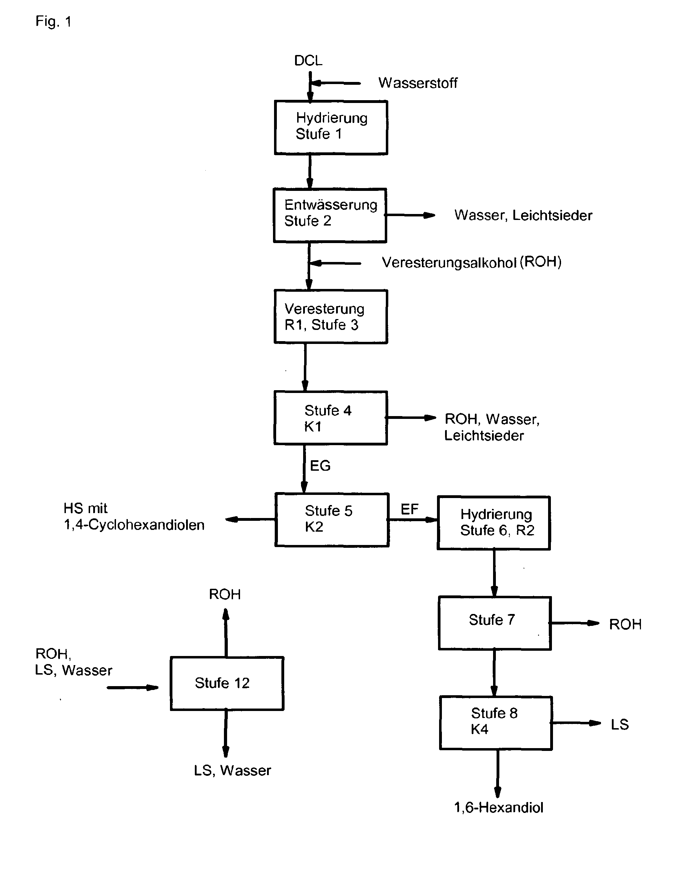

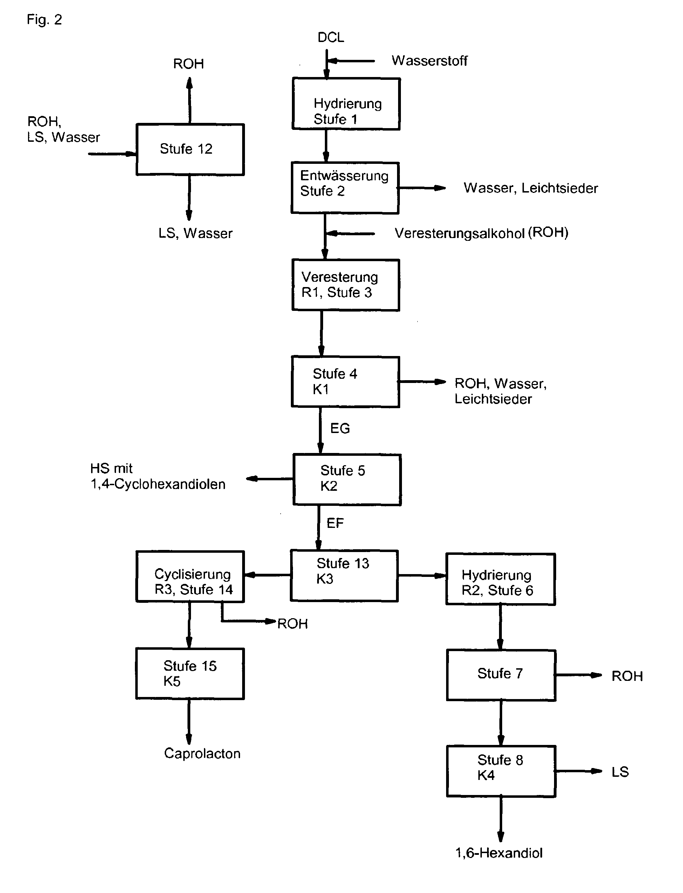

Wie in Fig. 1 (Vergleichsprozess) und Fig. 2 dargestellt, wird die Dicarbonsäurelösung (DCL) hydriert, nach Entwässerung, zusammen mit einem C₁- bis C₃-Alkohol, vorzugsweise Methanol, in den Veresterungsreaktor R₁ eingespeist, in dem die Carbonsäuren verestert werden. Das erhaltene Veresterungsgemisch gelangt dann in die Kolonne K₁, in der der überschüssige Alkohol (ROH), Wasser und Leichtsieder (LS) über Kopf abdestilliert und das Estergemisch (EG) als Sumpf abgezogen und in die Kolonne K₂ eingespeist wird. In dieser Kolonne wird das EG in eine von 1,4-Cyclohexandiolen überwiegend befreite (maximal noch 5 Gew.-%, bevorzugt unter 1 Gew.% der im Zulauf enthaltenen 1,4-Cyclohexandiole) Esterfraktion (EF) und eine Sumpffraktion bestehend aus Hochsiedern (HS) und cis- und trans-1,4-Cyclohexandiolen (1,4-CHDO) fraktioniert. Die Esterfraktion wird dann zu 1,6-Hexandiol und Veresterungsalkohol hydriert und in K4 reindestilliert, oder, sofern man Caprolacton gewinnen möchte, in eine weitere Fraktionierkolonne K₃ geleitet, in der die Esterfraktion in ein Kopfprodukt, enthaltend überwiegend (> 50 Gew.%) Adipinsäurediester (ADSE), vorzugsweise -dimethylester und ein Sumpfprodukt enthaltend mindestens 90 Gew.-% 6-Hydroxycapronsäureester (HCSE), vorzugsweise -methylester aufgetrennt wird. Die überwiegend Adipinsäurediester enthaltende Fraktion wird dann in der katalytischen Hydrierung R₂ zu 1,6-Hexandiol hydriert, das in der Kolonne K₄ reindestilliert wird.

Die 6-Hydroxycapronsäureesterfraktion wird im Reaktor R₃, wie in Fig. 2 beschrieben, einer thermischen Behandlung über 100°C, in der Regel 150 bis 350°C, vorzugsweise 200 bis 300°C bei vermindertem Druck, z.B. 900 bis 10 mbar, vorzugsweise 300 bis 20 mbar unterworfen; dies führt zur Cyclisierung des Esters unter Bildung von ε - Caprolacton, das in der Kolonne K₅ reindestilliert wird.

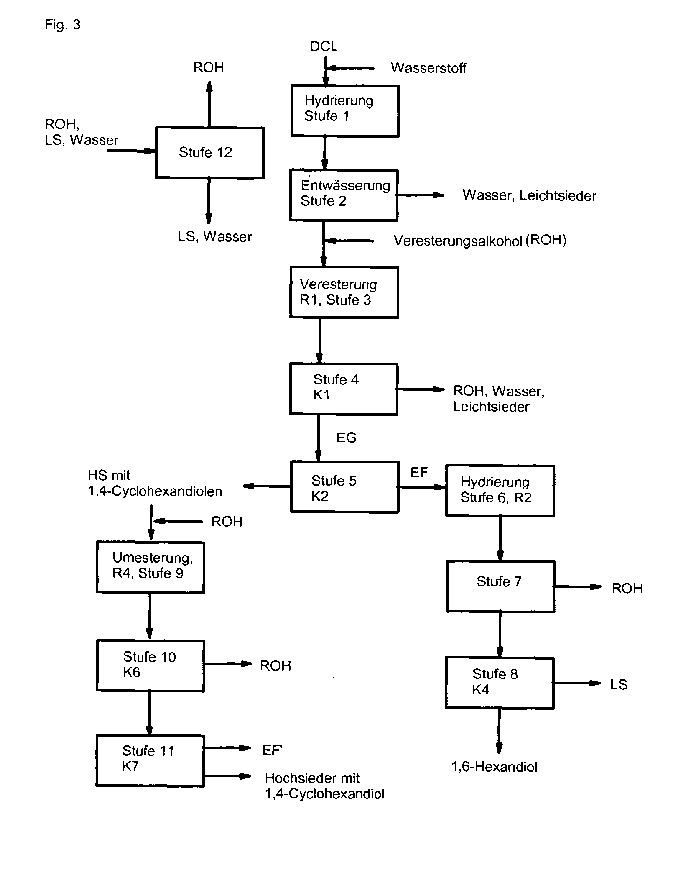

Zur Steigerung der Gesamtausbeute an C6-Wertprodukten, wie in Fig. 3 beschrieben, kann das in Kolonne 2 anfallende Hochsiedergemisch zusätzlich nochmals mit Veresterungsalkohol ROH umgesetzt (R4), anschließend in einer weiteren Kolonne K₆ von überschüssigem Alkohol ROH befreit und in einer Kolonne K7 in Hochsieder, die die 1,4-Cyclohexandiole enthalten und ein weiteres Estergemisch EF' aufgetrennt werden. Dieses EF' kann beispielsweise zusammen mit dem Estergemisch EG wieder in die Kolonne K₂ eingespeist werden.

Das erfindungsgemäße Verfahren wird im Folgenden im Einzelnen anhand der Fig. 2 und der einen Vergleichsprozess darstellenden Figuren 1 und 3 erläutert.

Die Verfahrensschritte sind in Stufen aufgeschlüsselt, wobei die Stufen 1,2, 3, 4, 5, 6, 7 und 8 sowie 12, 13, 14 und 15 für das Verfahren essentiell sind und die Stufen 4 und 5 sowie 7 und 8 auch zusammengefasst werden können. Die Stufen 9, 10 und 11 sind fakultativ, aber zur Erhöhung der Wirtschaftlichkeit des Verfahrens gegebenenfalls sinnvoll.

Für die katalytische Hydrierung der DCL in Schritt a) des erfindungsgemäßen Verfahrens (Stufe 1) dienen Katalysatoren, die mindestens ein Metall der 7. bis 12. Gruppe des Periodensystems, beispielsweise Ruthenium, Palladium, Platin, Nickel, Cobalt, Eisen, Rhenium, Iridium, Kupfer, Osmium und Zink enthalten.
Bevorzugt sind dabei die Metalle Ruthenium, Nickel, Cobalt, Rhenium und Kupfer. Diese Metalle können sowohl in Form der Metalle als auch deren Verbindungen, wie z.B. Oxide und Sulfide eingesetzt werden.

Bevorzugt sind weiterhin Gemische oder Legierungen aus mindestens zwei der Metalle der 7. bis 12. Gruppe des Periodensystems. Beispielhaft genannt seinen Palladium/Rhenium, Platin/Rhenium und Cobalt/Kupfer.

Gut geeignet sind weiterhin so genannte Vollkatalysatoren, die keine Träger enthalten und aus Metallen, Metalloxiden oder deren Gemischen bestehen. Bevorzugt sind dabei Eisen- und insbesondere Cobalt-Vollkatalysatoren.

Die Metalle oder Metallverbindungen können ohne Träger verwendet werden. Bevorzugt werden sie jedoch auf Träger, wie z. B. TiO₂, Al₂O₃, ZrO₂, SiO₂, HfO₂, Kohle, Zeolithe oder deren Gemische aufgebracht. Diese Trägerkatalysatoren können in verschiedensten Konfektionierungsformen, wie z. B. Strängen, Tabletten oder Ringen verwendet werden.

Kupfer, Nickel- und Cobalt können bevorzugt in Form von Raney-Nickel, Raney-Kupfer oder Raney-Cobalt eingesetzt werden. Auch die Raney-Katalysatoren können in allen bekannten Konfektionierungsformen, z. B. als Tabletten, Stränge oder Granulate, eingesetzt werden. Geeignete Raney-Kupfer-Katalysatoren sind z. B. Raneykupfer-Nuggets, die in WO-A 99/03.801 beschrieben sind.

Besonders geeignet für die Hydrierung der DCL ist weiterhin ein Katalysator, enthaltend auf Titandioxid-Formkörpern geträgertes Ruthenium, wobei die Titandioxid-Formkörper durch Behandeln von Titandioxid vor oder nach dem Formen zum Formkörper mit 0,1 bis 30 Gew.-% einer Säure, in der Titandioxid schwer löslich ist, erhalten werden.

Das katalytisch aktive Ruthenium wird nach an sich bekannten Verfahren, bevorzugt auf vorgefertigtes TiO₂ als Trägermaterial aufgebracht.

Ein für die Verwendung in dem Ruthenium enthaltenden Katalysator bevorzugt geeigneter Titandioxid-Träger kann entsprechend DE-A 197 38 464 durch Behandeln von Titandioxid vor oder nach dem Formen des Formkörpers mit 0,1 bis 30 Gew.-% einer Säure, bezogen auf Titandioxid, in der das Titandioxid schwer löslich ist, erhalten werden. Bevorzugt wird Titandioxid in der Anatas-Modifikation verwendet. Als derartige Säure sind beispielsweise Ameisensäure, Phosphorsäure, Salpetersäure, Essigsäure oder Stearinsäure geeignet.

Die Aktivkomponente Ruthenium kann in Form einer Rutheniumsalzlösung auf den so erhaltenen Titandioxidträger in einer oder mehreren Tränkstufen aufgebracht werden. Anschließend wird der getränkte Träger getrocknet und gegebenenfalls calciniert. Es ist jedoch auch möglich, Ruthenium aus einer Rutheniumsalzlösung, bevorzugt mit Natriumcarbonat, auf einen als Pulver in wässriger Suspension vorliegendes Titandioxids zu fällen. Die ausgefällten Niederschläge werden gewaschen, getrocknet, gegebenenfalls calciniert und verformt. Weiterhin können flüchtige Rutheniumverbindungen, wie beispielsweise Rutheniumacetylacetonat oder Rutheniumcarbonyl, in die Gasphase überführt werden und in an sich bekannter Weise auf den Träger aufgebracht werden, was als Chemical vapor deposition bezeichnet wird.

Andere bevorzugte Trägermaterialien sind Zirkonoxid, Siliziumcarbid und Kohle. Insbesondere Kohle (Aktivkohlen) haben den Vorteil des geringen Litergewichts bei gleichzeitig hoher Oberfläche und chemischer Resistenz gegenüber Säuren. Die Kohle-Träger können vor der Verwendung oxidativ mit z.B. Luft oder Salpetersäure vorbehandelt werden, ebenfalls geeignet ist die Behandlung mit starken Säuren wie Schwefelsäure, Salzsäure oder Phosphorsäure. Die Vorbehandlung führt im Allgemeinen zu höherer katalytsichen Aktivität.

Die so erhaltenen, geträgerten Katalysatoren können in allen bekannten Konfektionierungsformen vorliegen. Beispiele sind Stränge, Tabletten oder Granulate. Vor ihrer Verwendung werden die Rutheniumkatalysatorvorläufer durch Behandlung mit wasserstoffhaltigem Gas, bevorzugt bei Temperaturen über 100°C reduziert. Bevorzugt werden die Katalysatoren vor ihrem Einsatz im erfindungsgemäßen Verfahren bei Temperaturen von 0 bis 50°C, bevorzugt bei Raumtemperatur, mit sauerstoffhaltigen Gasgemischen, bevorzugt mit Luft-Stickstoffgemischen, passiviert. Es ist auch möglich, den Katalysator in oxidischer Form in den Hydrierreaktor einzubauen und unter Reaktionsbedingungen zu reduzieren.

Der erfindungsgemäß besonders bevorzugte Katalysator weist einen Rutheniumgehalt von 0,01 bis 10 Gew.-%, bevorzugt von 0,1 bis 6, bezogen auf das Gesamtgewicht des Katalysators aus katalytisch aktivem Metall und Träger, auf. Der erfindungsgemäße Katalysator kann einen Schwefelgehalt von 0,01 bis 1 Gew.-%, bezogen auf das Gesamtgewicht des Katalysators, aufweisen, wobei die Schwefel-Bestimmung coulometrisch erfolgt.

Die Rutheniumoberfläche beträgt dabei von 1 bis 20 m²/g, bevorzugt von 5 bis 15 m²/g und die BET-Oberfläche (bestimmt nach DIN 66 131) von 5 bis 500 m²/g, bevorzugt von 50 bis 200 m²/g.

Die erfindungsgemäßen Katalysatoren weisen ein Porenvolumen von 0,1 bis 100 ml/g auf. Weiterhin zeichnen sich die Katalysatoren durch eine Schneidhärte von 1 bis 100 N aus.

Die Hydrierkatalysatoren können im Reaktionsgemisch suspendiert sein. Bevorzugt ist, sie im Hydrierreaktor fest anzuordnen. Die Hydrierung kann diskontinuierlich oder bevorzugt kontinuierlich durchgeführt werden. Das Reaktionsgemisch kann dabei in Sumpf- oder Rieselfahrweise über den Katalysator geleitet werden.
Die Hydrierung kann in einem einzigen Reaktor oder in zwei hintereinander geschalteten Reaktoren durchgeführt werden. Verwendet man zwei Reaktoren, so können die beiden Reaktoren den gleichen Katalysator oder zwei verschiedene Katalysatoren enthalten. Dabei können sich die beiden Reaktoren in der Hydriertemperatur und dem Wasserstoffpartialdruck unterscheiden.

Es ist weiterhin möglich, die Hydrierung in einem einzigen Reaktor, der mit einem einzigen Katalysator gefüllt ist, so durchzuführen, dass die Hydriertemperatur im Reaktor innerhalb eines gewünschten Temperaturbereichs ansteigt. Dabei liegt der Temperaturbereich für die Hydrierung zwischen 50 und 200°C, bevorzugt sind 70 bis 180°C, besonders bevorzugt zwischen 90 und 160°C.

Der Reaktionsdruck, im Wesentlichen erzeugt durch Wasserstoff, liegt zwischen 5 und 35 bar.

Als Wasserstoff kann man reinen Wasserstoff verwenden, es ist aber auch möglich, technisch sogar bevorzugt, das Abgas aus einer anderen Hydrierung, beispielsweise der der Ester zu 1,6-Hexandiol, zur Hydrierung ganz oder zumindest teilweise zu verwenden.

Der molare Überschuss an Wasserstoff bezogen auf die zu hydrierende Komponente liegt zwischen 1 und 5000 Mol-%, bevorzugt sind 10 bis 3000 Mol-%, besonders bevorzugt sind 50 bis 1000 Mol-%.

Die Dicarbonsäurelösung (DCL) ist im Allgemeinen eine wässrige Lösung mit einem Wasseranteil von 20 bis 80 Gew.-%. Da eine Veresterungsreaktion eine Gleichgewichtsreaktion darstellt, bei der Wasser entsteht, ist es sinnvoll, insbesondere bei Veresterung mit z.B. Methanol, vorhandenes Wasser vor der Reaktion zu entfernen, vor allem wenn während der Veresterungsreaktion Wasser nicht, z.B. nicht azeotrop, entfernt werden kann. Die Entwässerung (Stufe 2) in Schritt b) kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von 1 bis 1 500 mbar, besonders bevorzugt 5 bis 1 100 mbar, ganz besonders bevorzugt 20 bis 1 000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, dass das Wasser säurefrei erhalten wird oder man kann die in der DCL enthaltenen niederen Monocarbonsäuren - im Wesentlichen Ameisensäure, wenn noch vorhanden - zum größten Teil mit dem Wasser abdestillieren, bevorzugt 60 - 95 Gew.-% der im Zulauf enthaltenen Säuren wie Ameisensäure und Essigsäure, damit diese in der Veresterung keinen Veresterungsalkohol binden. Zusammen mit dem Wasser können noch weitere Komponenten abgetrennt werden, z.B. Cyclohexanol, ggf. noch vorhandenen Cyclohexanon. Diese können z.B. durch Phasentrennung von Wasser getrennt werden und als Wertprodukte z.B. in die Cyclohexanol/Cyclohexanon-Gewinnung abgegeben werden.

Dem Carbonsäurestrom aus der Stufe 2 wird Alkohol ROH mit 1 bis 10 C-Atomen zugemischt. Dabei können Methanol, Ethanol, Propanol oder iso-Propanol oder Gemische der Alkohole, bevorzugt aber Methanol einerseits oder C₄ und höhere Alkohole, insbesondere mit 4 bis 8 C-Atomen und bevorzugt n- oder i-Butanol oder auch n-Pentanol oder i-Pentanol andererseits verwendet werden. Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 3, in dem die Carbonsäuren mit dem Alkohol verestert werden. Die Veresterungsreaktion kann bei 50 bis 400°C, bevorzugt 70 bis 300°C, besonders bevorzugt 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z.B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10. Dabei müssen nicht alle im System vorhandenen Carboxylgruppen als Ester des eingesetzten Alkohols vorliegen, sondern ein Teil kann in Form von dimeren oder oligomeren Estern mit dem OH-Ende der Hydroxycapronsäure vorliegen.

Das Veresterungsgemisch wird in Stufe 4 in eine Destillationskolonne eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder - Alkoholate, oder Amine in Substanz oder in dem Veresterungsalkohol gelöst verwendet. Es ist ebenfalls der Einsatz von Ionentauschern möglich, wobei diese bevorzugt durch Regeneration immer wieder einsetzbar sind.

Wird in Stufe 4 eine Kolonne verwendet, so erfolgt der Zulauf zur Kolonne bevorzugt zwischen dem Kopf- und dem Sumpfstrom. Über Kopf wird bei Drücken von 1 bis 1 500 mbar, bevorzugt 20 bis 1 000 mbar, besonders bevorzugt 40 bis 800 mbar und Temperaturen zwischen 0 und 150°C, bevorzugt 15 und 90°C und insbesondere 25 und 75°C der überschüssige Veresterungsalkohole ROH, Wasser sowie entsprechende Ester der Ameisensäure, Essigsäure und Propionsäure abgezogen. Dieser Strom kann entweder verbrannt oder bevorzugt in der Stufe 12 weiter aufgearbeitet werden.

Als Sumpf wird ein Estergemisch erhalten, das vorwiegend aus den Estern des eingesetzten Alkohols ROH mit Dicarbonsäuren wie Adipinsäure und Glutarsäure, Hydroxycarbonsäuren wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 4 Gew-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 4 wird in die Stufe 5 eingespeist. Dabei handelt es sich um eine Destillationskolonne, bei der der Zulauf zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1 000 mbar, bevorzugt 5 bis 500 mbar, besonders bevorzugt 10 bis 200 mbar betrieben.

Die Kopffraktion besteht überwiegend aus Restwasser und Restalkohol ROH, Estern des Alkohols ROH mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestern mit Dicarbonsäuren wie Adipinsäure, Glutarsäure und Bernsteinsäure, 1,2-Cyclohexandiolen, Caprolacton und Valerolaceton.

Die genannten Komponenten können zusammen über Kopf abgetrennt oder in einer weiteren bevorzugten Ausführungsform in der Kolonne der Stufe 5 in einen Kopfstrom, der überwiegend Restwasser und Restalkohol sowie die oben erwähnten Bestandteile mit 3 bis 5 C-Atomen enthält und einen Seitenstrom, der überwiegend die oben erwähnten Bestandteile der C₆-Ester enthält, aufgetrennt werden. Der die Ester der C₆-Säuren enthaltende Strom, entweder als Gesamt-Kopfstrom oder als Seitenstrom kann dann, je nachdem wieviel Caprolacton hergestellt werden soll, im Grenzfall - ohne Caprolacton-Herstellung - ganz in die Hydrierung (Stufe 6) gelangen, erfindungsgemäß jedoch zum Teil oder als Gesamtstrom in die Stufe 13 eingespeist werden.

Die schwersiedenden Komponenten des Stromes aus Stufe 4, überwiegend bestehend aus 1,4-Cyclohexandiolen oder deren Estern, dimeren oder oligomeren Estern sowie nicht näher definierte z.T. polymeren Bestandteilen der DCL, werden über den Abtriebsteil der Kolonne der Stufe 5 abgetrennt. Diese können zusammen anfallen oder so, dass über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sogenannten Umesterung in die Stufe 9 gelangen.

Die Stufen 4 und 5 können, insbesondere wenn nur kleinere Mengen verarbeitet werden, zusammengefasst werden. Dazu kann beispielsweise in einer absatzweise durchgeführten fraktionierten Destillation der C₆-Esterstrom gewonnen werden, wiederum ohne dass 1,4-Cyclohexandiole in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind in H. Kropf, Houben-Weyl, Methoden der Organischen Chemie, Band IV/1 c, Georg Thieme Verlag Stuttgart, 1980, S. 45 bis 67, und Beispiele für heterogene Katalysatoren sind in Houben-Weyl, Methoden der Organischen Chemie, Band IV/1c, S. 16 bis 26, beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, TiO₂, ZnO₂, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≙ 0, d > 0, a > b/2, b > a/4, a > c, a > d gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP 0 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so dass man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m²/g.

Weitere bevorzugte Hydrierkatalysatoren enthalten neben Cu noch La- und Al-Oxide. Sie sind beispielsweise in DE-A 10313702 beschrieben ist.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im Allgemeinen Temperaturen von 150 bis 300°C bei Drücken von 1 bis 100 bar, bevorzugt 15 bis 70 bar angewandt. Dabei wird zweckmäßig mindestens so viel Wasserstoff als Hydriermittel und Trägergas verwendet, dass Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im Allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinander geschalteten Reaktoren durchgeführt werden. Die Hydrierung in Flüssigphase über einem Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden. Kreisgas ist insbesondere bei Rieselfahrweise nicht notwendig.

Die Hydrierung kann diskontinuierlich, bevorzugt kontinuierlich erfolgen.

Der Hydrieraustrag besteht im Wesentlichen aus 1,6-Hexandiol und dem Alkohol ROH. Weitere Bestandteile sind vor allem, falls der gesamte leichtsiedende Strom der Stufe 5 eingesetzt wurde, 1,5-Pentandiol, 1,4-Butandiol, 1,2-Cyclohexandiole sowie kleine Mengen von Monoalkoholen mit 1 bis 6 C-Atomen, ggf. Ether und Wasser.

Der Hydrieraustrag wird in der Stufe 7 (eine Destillationskolonne), in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1 500 mbar, bevorzugt 30 bis 1 200 mbar, besonders bevorzugt 50 bis 1 000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 3 zurückgeführt werden oder in die Stufe 9 oder in die Stufe 12 gelangen.

Der 1,6-Hexandiol enthaltene Stoffstrom wird in der Stufe 8 in einer Kolonne gereinigt. Dabei werden 1,5-Pentandiol, die 1,2-Cyclohexandiole sowie weitere eventuell vorhandene Leichtsieder über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99,5 %, bevorzugt mindestens 99,7%, besonders bevorzugt über 99,9% aus einem Seitenstrom der Kolonne entnommen. Dabei werden bei Drücken von 1 bis 1 000 mbar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 7 und 8 auch in einer diskontinuierlichen fraktionierten Destillation zusammengefasst werden.

Um das erfindungsgemäße Verfahren möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH enthaltende Strom aus Stufe 4 und/oder 7 in der Stufe 12 aufgearbeitet werden. Dazu wird vorteilhaft eine Kolonne verwendet, in der Komponenten, die leichter sieden als der Alkohol ROH über Kopf, Wasser und Komponenten, die höher sieden als der Alkohol ROH, über Sumpf vom Alkohol ROH, der in einem Seitenstrom gewonnen wird, abgetrennt werden. Die Kolonne wird zweckmäßig bei 500 bis 5 000 mbar, bevorzugt bei 800 bis 3 000 mbar betrieben.

Nach einer weiteren bevorzugten Ausführungsform des erfindungsgemäßen Verfahrens wird der hochsiedende Strom aus Stufe 5 zur Erhöhung der Gesamtausbeute an Wertprodukten, bezogen auf eingesetzte DCL aufgearbeitet. Dazu wird in der Stufe 9 der Anteil an dimeren und oligomeren Estern der Adipinsäure bzw. Hydroxycapronsäure mit weiteren Mengen des Alkohols ROH, vorzugsweise Methanol, in Gegenwart eines Katalysators umgesetzt. Das Gewichtsverhältnis von Alkohol ROH und dem Sumpfstrom aus Stufe 5 beträgt zwischen 0,1 bis 20, bevorzugt 0,5 bis 10, besonders bevorzugt 1 bis 5. Als Katalysatoren eignen sich prinzipiell die bereits für die Veresterung in Stufe 3 beschrieben. Bevorzugt werden jedoch Lewissäuren oder Lewisbasen eingesetzt. Beispiele hierzu sind Verbindungen oder Komplexe des Aluminiums, Zinns, Antimons, Zirkons oder Titans, wie Zirkoniumacetylacetonat oder Tetraalkyltitanat wie Tetraisopropyltitanat, die in Konzentrationen von 1 bis 10 000 ppm, bevorzugt 50 bis 6 000 ppm, besonders bevorzugt 100 bis 4 000 ppm, angewandt werden. Besonders bevorzugt sind Titanverbindungen.

Die Umesterung kann absatzweise oder kontinuierlich, in einem Reaktor oder mehreren Reaktoren, in Reihe geschalteten Rührkesseln oder Rohrreaktoren bei Temperaturen zwischen 100 und 300°C, bevorzugt 120 bis 270°C, besonders bevorzugt 140 bis 240°C und den sich dabei einstellenden Eigendrücken, durchgeführt werden. Die benötigten Verweilzeiten liegen bei 0,5 bis 10 Stunden, bevorzugt bei 1 bis 4 Stunden.

Dieser Strom aus der Stufe 9 lässt sich im Falle der Veresterung mit Methanol z.B. wieder in die Stufe 4 einschleusen. Zur Vermeidung von Aufpegelungen, vor allem von 1,4-Cyclohexandiolen, muss dann absatzweise oder kontinuierlich ein Teilstrom der Hochsieder aus Stufe 5 ausgeschleust werden. Eine andere Möglichkeit ist, den Strom aus Stufe 9 nicht in Stufe 4 zurückzuführen, sondern ihn, analog zur Stufe 4, in einer Stufe 10 in vorwiegend Alkohol ROH, der dann wieder in die Stufe 3, 9 oder 12 gelangen kann und einen Strom der die Ester enthält, aufzutrennen.

Dieser Esterstrom kann prinzipiell (mit der Maßgabe der Vermeidung von Aufpegelungen der 1,4-Cyclohexandiole) in die Stufe 5 zurückgeführt werden oder wird bevorzugt in eine weitere Stufe 11 in die Ester der C₆-Säuren und, mengenmäßig eher unbedeutend, in die Ester der C₅-Säuren einerseits, die entweder in die Stufe 5 oder direkt in die Stufe 6 eingeschleust werden und Hochsieder andererseits, die 1,4-Cyclohexandiole enthalten, aufgetrennt, worauf die Hochsieder ausgeschleust werden.

Für die Caprolactonherstellung wird der vorwiegend Ester der C₆-Säuren enthaltende Strom aus der Stufe 5 eingesetzt. Dazu wird dieser Strom in Stufe 13, einer Destillationskolonne, in einen überwiegend Adipinsäurediester enthaltenden Strom, der die vorhandenen 1,2-Cyclohexandiole enthält, über Kopf und einen überwiegend 6-Hydroxycapronsäureester enthaltenden Strom, der keine 1,2-Cyclohexandiole enthält, über Sumpf aufgetrennt. Die Kolonne wird bei Drücken von 1 bis 500 mbar, bevorzugt 5 bis 350 mbar, besonders bevorzugt 10 bis 200 mbar und Sumpftemperaturen von 80 bis 250°C, bevorzugt 100 bis 200°C, besonders bevorzugt 110 bis 180°C betrieben. Die Kopftemperaturen stellen sich dabei entsprechend ein.

Wichtig für eine hohe Reinheit und hohe Ausbeute an Caprolacton ist die Abtrennung der 1,2-Cyclohexandiole vom Hydroxycapronsäureester, da diese Komponenten Azeotrope miteinander bilden.

Zur Verminderung des Trennaufwands kann es vorteilhaft sein, in der Stufe 13 zusammen mit dem Adipinsäurediester auch etwas Hydroxycapronsäureester abzutrennen. Die Gehalte an Hydroxycapronsäureester liegen dabei vorteilhaft zwischen 0,2 und 15 Gew.-%. Je nach Alkoholkomponente der Ester wird dieser Anteil Hydroxycapronsäureester zusammen mit dem Adipinsäurediester über Kopf (z.B. Methylester) oder über Sumpf (z.B. Butylester) abgetrennt.

Der 6-Hydroxycapronsäureester enthaltende Sumpfstrom der Stufe 13 wird in der Stufe 14 in an sich bekannter Weise entweder in der Gas- oder Flüssigphase zu Alkohol und Caprolacton umgesetzt. Bevorzugt ist die Flüssigphase.

Die Reaktion wird ohne Katalysator oder aber bevorzugt in Anwesenheit eines Katalysators durchgeführt. Als Katalysatoren eignen sich saure oder basische Katalysatoren, die homogen gelöst oder heterogen vorliegen können. Beispiele sind Alkali- und Erdalkalimetallhydroxide-, -oxide, -carbonate, -alkoxylate oder -carboxylate, Säuren wie Schwefel- oder Phosphorsäure, organische Säuren wie Sulfonsäuren oder Mono- oder Dicarbonsäuren, bzw. Salze der vorgenannten Säuren, Lewissäuren oder Lewisbasen, bevorzugt aus der III. und IV. Hauptgruppe bzw. der I. bis VIII. Nebengruppe des Periodensystems der Elemente.

Bevorzugt verwendet man die gleichen Katalysatoren, die auch in der Stufe 9 eingesetzt werden, da der hochsiedende Ausschleusstrom der Stufe 14 oligomere Hydroxycapronsäureeinheiten enthält, die vorteilhaft über die Stufe 9 wiederverwertet werden können. Wird ein heterogener Katalysator eingesetzt, so beträgt die Katalysatorbelastung üblicherweise 0,05 bis 5 kg Edukt/I Katalysator und Stunde. Bei homogenen Katalysatoren wird der Katalysator bevorzugt dem Eduktstrom zugemischt. Dabei beträgt die Konzentration üblicherweise 10 bis 10000 ppm, bevorzugt 50 bis 5000 ppm, besonders bevorzugt 100 bis 1000 ppm. Die Reaktion wird üblicherweise bei 150 bis 400°C, bevorzugt 180 bis 350°C, besonders bevorzugt 190 bis 280°C und Drücken von 1 bis 1020 mbar, bevorzugt 5 bis 500 mbar, besonders bevorzugt 10 bis 200 mbar durchgeführt.

In manchen Fällen ist es vorteilhaft, die Cyclisierungsreaktion in Gegenwart von hochsiedenden Mono-, Di- oder Polyolen wie z.B. Decanol, Undecanol, Tridecanol, Pentadecanol, Octadecanol, 1,4-Butandiol, 1,5-Pentandiol, 1,6-Hexandiol, 1,4-Cyclohexandiolen, Butylethylpropandiol, Neopentylglycol, Triethylenglykol, Tetraethylenglykol, Trimethylolpropan oder Glycerin durchführen.

Diese hochsiedenden Alkohole oder Polyole werden vorgelegt und/oder dem Reaktionsgemisch in Konzentrationen bis 5 Gew.-% zugesetzt oder separat zudosiert z.B. jeweils in Konzentrationen von 1 und 20000 ppm, bevorzugt 10 bis 4000 ppm, besonders bevorzugt 50 bis 2000 ppm.

Wird die Cyclisierung in der Flüssigphase durchgeführt, werden die Reaktionsprodukte, vorwiegend Veresterungsalkohol ROH und Caprolacton gasförmig aus dem Reaktionsgemisch entfernt. Vorteilhaft ist eine dem Reaktionsgefäß aufgesetzte Kolonne, in der noch nicht umgesetztes Edukt im Reaktionssystem gehalten werden kann und über Kopf der Alkohol und Caprolacton abgezogen werden. Dabei kann die Kondensation des Produktstroms so erfolgen, dass fraktioniert kondensiert wird, d.h. zuerst vorwiegend Caprolacton, dann der Veresterungsalkohol. Selbstverständlich kann auch nur der Alkohol über Kopf, Caprolacton dagegen in einem Seitenstrom gewonnen werden. Der Alkohol-Strom kann vorteilhaft in die Stufe 3, 9 oder 12 zurückgeführt werden. Das Sumpfprodukt der Cyclisierung kann in die Stufe 9 eingeschleust werden.

Der Zulauf zum Reaktionsgefäß kann ohne Vorheizung erfolgen. Werden homogene Katalysatoren verwendet, so ist es vorteilhaft, den Eduktstrom direkt in den Cyclisierungssumpf einzutragen. Dabei kann der Katalysator entweder schon vor der Reaktion dem Feed zugefügt werden, oder direkt in das Reaktionsgefäß gegeben werden.

Vorteilhafter ist es jedoch den Zulauf vorzuheizen, vor allem, wenn der Katalysator bereits gelöst ist und ein Hydroxycapronsäureester mit einer C₁-C₅-Alkoholkomponente verwendet wird. Die Vorheiztemperatur liegt dabei zwischen 100 und 300°C, bevorzugt bei 130 - 270°C, besonders bevorzugt bei 150 - 250°C. Bei diesen Temperaturen reagiert der Hydroxycapronsäureester bereits zum Teil zu Alkohol, Caprolacton und dimeren bzw. oligomeren Hydroxycapronsäureestern. Dies bewirkt, dass nur wenig Hydroxycapronsäureester, wenn er in das heiße Reaktionsgefäß gelangt, sofort aus dem Reaktionssumpf abdestillieren kann. Auf diese Weise werden Kolonnenböden eingespart.

Eine weitere vorteilhafte Möglichkeit besteht darin, den überwiegenden Teil des Esteralkohols vor der Aufarbeitung des Caprolactons zu gewinnen, vor allem wenn dieser Alkohol, wie Methanol, niedrig siedet und in der Folge nur aufwändig kondensierbar wäre. Dazu wird der Hydroxycapronsäuremethylester in Gegenwart eines Katalysators wie oben beschrieben vorerhitzt, wobei bereits der freiwerdende Alkohol abdestilliert. Dies geschieht vorteilhaft bei 100-1100 mbar, einem Druckbereich, bei dem der Esteralkohol leicht kondensierbar ist. Dieses Vorgehen ist auch in Gegenwart der oben beschriebenen hochsiedenden Alkohole möglich.

Der Caprolacton-Produktstrom der Stufe 14 wird in der Stufe 15 weiter aufgearbeitet. Dabei kann es sich um eine oder mehrere Kolonnen handeln. Wird eine Kolonne verwendet, so werden über Kopf der gegebenenfalls noch vorhandene Veresterungsalkohol, sowie andere C₁- bis C₆-Leichtsieder abgetrennt, über Seitenstrom reines Caprolacton und über den Sumpf gegebenenfalls noch nicht umgesetzter Hydroxycapronsäureester, der zurückgeführt wird.

Hochreines Caprolacton erhält man, wenn in der Stufe 15 die erwähnten Leichtsieder in einer ersten Kolonne über Kopf abgezogen, Caprolacton und andere Hochsieder über Sumpf abgezogen, in eine zweite Kolonne eingespeist werden, wo Caprolacton über Kopf abgezogen wird. Handelt es sich bei dem zu gewinnenden Caprolactonstrom nur um kleinere Mengen, so kann Caprolacton mit einer Kolonne durch absatzweise fraktionierte Destillation gewonnen werden.

Die Destillationen werden bei Sumpftemperaturen von 70 bis 250°C, bevorzugt 90 bis 230°C, besonders bevorzugt 100 bis 210°C und Drücken von 1 bis 500 mbar, bevorzugt 5 bis 200 mbar, besonders bevorzugt 10 bis 150 mbar durchgeführt.

Nach dem erfindungsgemäßen Verfahren können Ausbeuten an 1,6-Hexandiol und Caprolacton von jeweils über 95 %, bei Reinheiten von über 99 % erzielt werden.

Das Verfahren wird anhand der nachfolgenden Beispiele näher erläutert, aber dadurch in keiner Weise eingeschränkt. Die Angaben über die Zusammensetzung der Stoffströme sind durch Gaschromatographie ermittelte Gew.%.

### Beispiel 1 (Vergleichsbeispiel ohne Hydrierung der DCL)

### Stufe 2: (Entwässerung)

0,1 kg Dicarbonsäurelösung/h (Adipinsäure, 6-Hydroxycapronsäure, 6-Oxocapronsäure, 1,4-Cyclohexandiole, 4-Hydroxycyclohexanon, Glutarsäure, 5-Hydroxyvaleriansäure, Ameisensäure, Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg erhalten mit einem Ameisensäuregehalt im Wasser von ca. 3 %. Im Sumpfstrom (5,5 kg) betrug der Wassergehalt ca. 0,4 %.

### Stufe 3: (Veresterung)

5,5 kg des Sumpfstroms aus Stufe 2 wurde mit 8,3 kg Methanol und 14 g Schwefelsäure umgesetzt. Die Säurezahl des Austrags abzüglich Schwefelsäure betrug ca. 10 mg KOH/g.

### Stufe 4:

In einer Kolonne wurden der Veresterungsstrom aus Stufe 3 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 5: (1,4-Cyclohexandiolabtrennung)

In einer 50 cm Füllkörperkolonne wurde der Sumpfstrom aus Stufe 4 fraktioniert destilliert (10 mbar, 75-90°C Kopftemperatur, bis 200°C Sumpftemperatur). Im Sumpf fanden sich die 1,4-Cyclohexandiole.

Als Leichsieder wurden 0,3 kg abdestilliert (Bernsteinsäuredimethylester, Valeriansäuremethylester, Pentansäuremethylester, Capronsäuremethylester, 1,2-Cyclohexandiole, Valerolacton, 5-Hydroxyvaleriansäuremethylester, Glutarsäuredimethylester, u.a.); als überwiegend Adipinsäuredimethylester und 6-Hydroxycapronsäuremethylester enthaltende Fraktion wurden 4,6 kg erhalten, die außerdem noch zwischen 2 bis 5 % Glutarsäuredimethylester und 5-Hydroxyvaleriansäuremethylester, zwischen 0,2 und 1 % Valerolacton, Caprolacton, 6,6-Dimethoxycapronsäuremethylester und 4-Hydroxycyclohexanon enthielten.

### Stufe 6: (Hydrierung Teilstrom)

2,7 kg C₆-Estergemisch aus Stufe 5 wurden kontinuierlich in einem 25 ml Reaktor an einem Katalysator hydriert (Katalysator, 70 Gew.-%, CuO, 25 Gew.-% ZnO, 5 Gew.-% Al₂O₃, der zuvor im Wasserstoffstrom bei 180°C aktiviert worden ist, Hydrierbedingungen: Zulauf 20 g/h, keim Umlauf, 220 bar, 220°C). Der Ester-Umsatz betrug 99,5 %, die 1,6-Hexandiolsetektivität betrug über 99 %.

### Stufe 7 und 8: (Hexandiolreinigung)

2,5 kg des Hydrieraustrags aus Stufe 6 wurden fraktioniert destilliert (Destillationsblase mit aufgesetzter 70 cm Füllkörperkolonne, Rücklaufverhältnis 2). Bei 1013 mbar wurden 0,5 kg Methanol abdestilliert und nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,6 % ab. Im Hexandiol befanden sich neben mengenmäßig unbedeutenden Komponenten noch ca. 0,2 % 1,4-Cyclohexandiole sowie ca. 0,02 % 6-Methoxyhexan-1-ol und 0,1 % 6,6-Dimethoxyhexan-1-ol.

### Stufe 13: (Trennung Adipinsäureester von 6-Hydroxycapronsäureester)

Von 2,0 kg Estergemisch aus Stufe 5 wurden in einer 3 I Destillationsblase mit aufgesetzter Kolonne (50 cm, 5 mm V2A-Metallringfüllkörper) und Rücklaufteiler bei 10 mbar vorwiegend Adipinsäuredimethylester abdestilliert (Rücklaufverhältnis 5, Kopftemperatur bis 100°C, Sumpftemperatur bis 120°C). Im Sumpf blieben 0,5 kg Hydroxycapronsäuremethylester (82 %ig, Rest vorwiegend dimerer Hydroxycapronsäuremethylester, kein Adipinsäuredimethylester) zurück.

### Stufe 14: (Cyclisierung)

In einer 250 ml Destillationsblase mit außenliegender Heizung und aufgesetzter Kolonne (70 cm, 5 mm V2A-Metallringfüllkörper) mit Rücklaufteiler wurden 10 g 1,6-Hexandiol mit Zusatz von 3000 ppm Tetraisopropyltitanat vorgelegt, auf 200°C bei 40 mbar aufgeheizt und stündlich 35 ml Sumpfprodukt aus Stufe 13, dem 1000 ppm Tetraisopropyltitanat sowie 200 ppm 1,6-Hexandiol zugesetzt wurden, zugefahren. Bei einer Kopftemperatur von 123 bis 124°C und einem Rücklaufverhältnis von 4 wurden bei 25°C vorwiegend Caprolacton, bei -78°C Methanol kondensiert.

### Stufe 15: (Caprolactonreinigung)

In einer 250 ml Destillationsblase mit aufgesetzter Kolonne (70 cm, 5 mm V2A-Metallringfüllkörper) und Rücklaufteiler (Rücklaufverhältnis 4) wurde das aus Stufe 14 erhaltene Caprolacton fraktioniert bei 40 mbar destilliert. Nach Abtrennung von im Wesentlichen Valerolacton (Sdp. 90 bis 110°C) wurde Caprolacton (Sdp. 131°C) in einer Reinheit (GC-Flächen-%) von 99,9 % erhalten.

### Beispiel 2: (erfindungsgemäßes Beispiel)

### Stufe 1: (DCL-Hydrierung)

0,1 kg/h Dicarbonsäurelösung wurden in einem Rohrreaktor (1 m lang, Inhalt 100 ml) bei 120°C und 20 bar Wasserstoffdruck, 25 Normliter Wasserstoff/h an 100 ml eines Ru (5 %)/Titandioxid-Katalysators hydriert. Die Hydrierung wurde 500 h betrieben, ohne dass sich die Zusammensetzung des Hydrieraustrages nennenswert änderte. Der Gehalt an 1,6-Hexandiol nach der Hydrierung lag um weniger als 0,1 % höher als vor der Hydrierung.

### Stufe 2: (Entwässerung)

0,1 kg/h Dicarbonsäurelösung aus Stufe 1 (Adipinsäure, 6-Hydroxycapronsäure, 1,4-Cyclohexandiole, Glutarsäure, 5-Hydroxyvaleriansäure, Ameisensäure, Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,04 kg erhalten mit einem Ameisensäuregehalt im Wasser von ca. 0,2 %. Im Sumpfstrom (5,5 kg) betrug der Wassergehalt ca. 0,4 %.
Der Vergleich zwischen dem Vergleichsbeispiel und dem erfindungsgemäßen Beispiel zeigt eine deutlich geringeren Mengen an Ameisensäure auf, wodurch ein reineres Endprodukt (siehe Stufe 7/8) als im Stand der Technik bekannt, erhalten wird.

### Stufe 3: (Veresterung)

5,5 kg des Sumpfstroms aus Stufe 2 wurde mit 8,3 kg Methanol und 14 g Schwefelsäure umgesetzt. Die Säurezahl des Austrags abzüglich Schwefelsäure betrug ca. 10 mg KOH/g.

### Stufe 4:

In einer Kolonne wurden der Veresterungsstrom aus Stufe 3 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 5: (1,4-Cyclohexandiolabtrennung)

In einer 50 cm Füllkörperkolonne wurde der Sumpfstrom aus Stufe 4 fraktioniert destilliert (10 mbar, 75-90°C Kopftemperatur, bis 200°C Sumpftemperatur). Im Sumpf fanden sich die 1,4-Cyclohexandiole.

Als Leichtsieder wurden 0,3 kg abdestilliert (Bernsteinsäuredimethylester, Valeriansäuremethylester, Pentansäuremethylester, Capronsäuremethylester, 1,2-Cyclohexandiole, Valerolacton, 5-Hydroxyvaleriansäuremethylester, Glutarsäuredimethylester, u.a.); als überwiegend Adipinsäuredimethylester und 6-Hydroxycapronsäuremethylester enthaltende Fraktion wurden 5,5 kg erhalten, die außerdem noch zwischen 2 bis 5 % Glutarsäuredimethylester und 5-Hydroxyvaleriansäuremethylester, zwischen 0,2 und 1 % Valerolacton und Caprolacton enthielten.

### Stufe 6: (Hydrierung Teilstrom)

3 kg C₆-Estergemisch aus Stufe 5 wurden kontinuierlich in einem 25 ml Reaktor an einem Katalysator hydriert (Katalysator, 70 Gew.-%, CuO, 25 Gew.-% ZnO, 5 Gew.-% Al₂O₃, der zuvor im Wasserstoffstrom bei 180°C aktiviert worden ist, Hydrierbedingungen: Zulauf 20 g/h, keim Umlauf, 220 bar, 220°C). Der Ester-Umsatz betrug 99,5 %, die 1,6-Hexandiolselektivität betrug über 99 %.

### Stufe 7 und 8: (Hexandiolreinigung)

2,9 kg des Hydrieraustrags aus Stufe 6 wurden fraktioniert destilliert (Destillationsblase mit aufgesetzter 70 cm Füllkörperkolonne, Rücklaufverhältnis 2). Bei 1013 mbar wurden 0,6 kg Methanol abdestilliert und nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,93 % ab. Im Hexandiol befanden sich neben mengenmäßig unbedeutenden Komponenten nur noch ca. 0,01 % 1,4-Cyclohexandiole. 6-Methoxyhexan-1-ol und 6,6-Dimethoxyhexan-1-ol wurden nicht gefunden.

### Stufe 13: (Trennung Adipinsäureester von 6-Hydroxycapronsäureester)

Von 2,5 kg Estergemisch aus Stufe 5 wurden in einer 3 I Destillationsblase mit aufgesetzter Kolonne (50 cm, 5 mm V2A-Metallringfüllkörper) und Rücklaufteiler bei 10 mbar vorwiegend Adipinsäuredimethylester abdestilliert (Rücklaufverhältnis 5, Kopftemperatur bis 100°C, Sumpftemperatur bis 120°C). Im Sumpf blieben 0,5 kg Hydroxycapronsäuremethylester (82 %ig, Rest vorwiegend dimerer Hydroxycapronsäuremethylester, kein Adipinsäuredimethylester) zurück.

### Stufe 14: (Cyclisierung)

In einer 250 ml Destillationsblase mit außenliegender Heizung und aufgesetzter Kolonne (70 cm, 5 mm V2A-Metallringfüllkörper) mit Rücklaufteiler wurden 10 g 1,6-Hexandiol mit Zusatz von 3000 ppm Tetraisopropyltitanat vorgelegt, auf 200°C bei 40 mbar aufgeheizt und stündlich 35 ml Sumpfprodukt aus Stufe 13, dem 1000 ppm Tetraisopropyltitanat sowie 200 ppm 1,6-Hexandiol zugesetzt wurden, zugefahren. Bei einer Kopftemperatur von 123 bis 124°C und einem Rücklaufverhältnis von 4 wurden bei 25°C vorwiegend Caprolacton, bei -78°C Methanol kondensiert.

### Stufe 15 : (Caprolactonreinigung)

In einer 250 ml Destillationsblase mit aufgesetzter Kolonne (70 cm, 5 mm V2A-Metallringfüllkörper) und Rücklaufteiler (Rücklaufverhältnis 4) wurde das aus Stufe 14 erhaltene Caprolacton fraktioniert bei 40 mbar destilliert. Nach Abtrennung von im Wesentlichen Valerolacton (Sdp. 90 bis 110°C) wurde Caprolacton (Sdp. 131°C) in einer Reinheit (GC-Flächen-%) von 99,9 % erhalten.

### Beispiel 3:

Beispiel 2, Stufe 1 wurde wiederholt, mit dem Unterschied, dass als Katalysator Ru (0,5 %) auf Aktivkohle eingesetzt wurde. Das Hydrierergebnis war äquivalent zu Beispiel 2.

### Beispiel 4:

Beispiel 2, Stufe 1 wurde wiederholt, mit dem Unterschied, dass als Katalysator Ni (10 % auf Aktivkohle bei 150 °C und 50 bar eingesetzt wurde. Das Hydrierergebnis war äquivalent zu Beispiel 2.

### Beispiel 5:

Beispiel 2, Stufe 1 wurde wiederholt, mit dem Unterschied, dass als Katalysator Co (10 % auf Aktivkohle bei 120°C und 50 bar eingesetzt wurde. Das Hydrierergebnis war äquivalent zu Beispiel 2.

## Patentansprüche

1. Verfahren zur Herstellung von 1,6-Hexandiol und ε-Caprolacton aus einem Adipinsäure, 6-Hydroxycapronsäure, 6-Oxocapronsäure, 4-Hydroxycyclohexanon, Ameisensäure und bezogen auf die Summe an Adipinsäure und Hydroxycapronsäure zwischen 0,5 und 5 Gew.- % 1,4-Cyclohexandiole enthaltenden Carbonsäuregemisch, das als Nebenprodukt der katalytischen Oxidation von Cyclohexan zu Cyclohexanon/Cyclohexanol mit Sauerstoff oder Sauerstoff enthaltenden Gasen durch Wasserextraktion des Reaktionsgemisches erhalten wird, durch Veresterung und Hydrierung eines Teilstroms zu 1,6-Hexandiol und Cyclisierung von 6-Hydroxycapronsäureester zu ε-Caprolacton, **dadurch gekennzeichnet, dass** man
a) nur die im wässrige Carbonsäuregemisch enthaltenen Aldehyde und Ketone bei einer Hydrierungstemperatur von 50 bis 200°C und einem Reaktionsdruck von 5 bis 35 bar absolut katalytisch zu den entsprechenden Alkoholen hydriert und mehr als 50 % der im Gemisch enthaltenen Ameisensäure abbaut,
b) die in dem wässrigen Reaktionsgemisch enthaltenen Mono- und Dicarbonsäuren nach Entwässerung mit einem niedermolekularen Alkohol zu den entsprechenden Carbonsäureestern umsetzt,
c) das erhaltene Veresterungsgemisch in einer ersten Destillationsstufe von überschüssigem Alkohol und Leichtsiedern befreit,
d) aus dem Sumpfprodukt in einer zweiten Destillationsstufe eine Auftrennung in eine von 1,4-Cyclohexandiolen abgereicherte Esterfraktion und eine 1,4-Cyclohexandiole enthaltende Fraktion durchführt,
e) von der Esterfraktion in einer dritten Destillationsstufe zumindest teilweise einen überwiegend 6-Hydroxycapronsäureester enthaltenden Strom abtrennt,
f) die Esterfraktion aus (e), aus der zumindest teilweise 6-Hydroxycapronsäureester entfernt wurde, katalytisch hydriert und durch Destillation des Hydrierproduktes in an sich bekannter Weise 1,6-Hexandiol gewinnt und
g) den überwiegend 6-Hydroxycapronsäureester enthaltenden Strom auf Temperaturen über 200°C bei vermindertem Druck erhitzt, wodurch 6-Hydroxycapronsäureester zu ε-Caprolacton cyclisiert wird und aus dem Cyclisierungsprodukt durch Destillation reines ε-Caprolacton gewinnt.

2. Verfahren gemäß Anspruch 1, **dadurch gekennzeichnet, dass** der Katalysator für die Hydrierung in Schritt a) mindestens ein Metall ausgewählt aus der Gruppe bestehend aus Ruthenium, Nickel, Kobalt, Rhenium und Kupfer, in metallischer Form oder als deren Verbindung enthält.

3. Verfahren gemäß Anspruch 1, **dadurch gekennzeichnet, dass** der Katalysator für die Hydrierung in Schritt a) auf Titandioxid- oder Aktivkohleformkörper geträgertes Ruthenium, Kobalt oder Nickel enthält.

4. Verfahren gemäß Anspruch 3, **dadurch gekennzeichnet, dass** der Katalysator für die Hydrierung in Schritt a) einen Metallgehalt im Bereich von 0,01 bis 10 Gew.-% bezogen auf das Gesamtgewicht des Katalysators aus katalytisch aktiven Metallen und Träger und eine BET-Oberfläche im Bereich von 5 bis 500 m²/g gemessen nach DIN 66 131, aufweist.

5. Verfahren gemäß einem der Ansprüche 1 bis 4, **dadurch gekennzeichnet, dass** der Katalysator für die Hydrierung in Schritt a) Ruthenium enthält.

6. Verfahren gemäß Anspruch 1, **dadurch gekennzeichnet, dass** man die Veresterung mit Alkoholen mit 1 bis 3 Kohlenstoffatomen durchführt.

7. Verfahren gemäß Anspruch 1, **dadurch gekennzeichnet, dass** man die Veresterung mit Alkoholen mit 4 bis 10 Kohlenstoffatomen durchführt.

8. Verfahren gemäß Anspruch 1, **dadurch gekennzeichnet, dass** man die Veresterung mit Methanol durchführt und in der Destillationsstufe (c) eine im wesentlichen von 1,4-Cyclohexandiolen freie Carbonsäuremethylesterfraktion am Kopf der Kolonne und eine die Hochsieder und die 1,4-Cyclohexandiole enthaltende Sumpffraktion gewinnt und die Carbonsäuremethylesterfraktion in die dritte Destillationsstufe (d) überführt.

9. Verfahren gemäß Anspruch 1, **dadurch gekennzeichnet, dass** man die Veresterung mit n- oder i-Butanol durchführt und in der Destillationsstufe (c) die 1,4-Cyclohexandiole mit den Leichtsiedern über Kopf abtrennt und die Carbonsäurebutylester als Seitenstrom oder als diese enthaltenden Sumpf gewinnt und in die dritte Destillationsstufe (d) überführt.

10. Verfahren gemäß Anspruch 1, **dadurch gekennzeichnet, dass** man die Destillationsstufe (c) und (d) in einer einzigen Kolonne durchführt.

11. Verfahren gemäß Anspruch 8, **dadurch gekennzeichnet, dass** man im Falle der Veresterung mit Methanol in einem oberen Seltenabzug eine im wesentlichen Dicarbonsäuremethylester enthaltende Fraktion, als unteren Seitenabzug eine im wesentlichen 6-Hydroxycapronsäuremethylesterfraktion und als Sumpfprodukt eine die 1,4-Cyclohexandiole enthaltende Fraktion abtrennt

12. Verfahren gemäß Anspruch 10, **dadurch gekennzeichnet, dass** man im Falle der Veresterung mit n- oder i-Butanol in einem oberen Seitenabzug eine im wesentlichen 6-Hydroxycapronsäurebutylester enthaltende Fraktion, als unteren Seitenabzug eine im Wesentlichen Dicarbonsäurebutylester enthaltende Fraktion und als Kopfprodukt eine die 1,4-Cyclohexandiole enthaltende Fraktion gewinnt.

13. Verfahren gemäß Anspruch 1, **dadurch gekennzeichnet, dass** man das Sumpfprodukt der Stufe (c) zumindest teilweise einer erneuten Veresterung unter weiterem Zusatz des niedermolekularen Alkohols und eines Veresterungskatalysators unterwirft und in einer getrennten Destillationsstufe analog (b) und (c) auftrennt oder erst nach Abtrennung der 1,4-Cyclohexandiole die erneute Veresterung durchführt und die Carbonsäureester enthaltende Fraktion in die Hydrierstufe (d) einleitet.

## Claims

1. A process for preparing 1,6-hexanediol and ε-caprolactone from a carboxylic acid mixture which comprises adipic acid, 6-hydroxycaproic acid, 6-oxocaproic acid, 4-hydroxycyclohexanone, formic acid and, based on the sum of adipic acid and hydroxycaproic acid, between 0.5 and 5% by weight of 1,4-cyclohexanediols, and is obtained as a byproduct of the catalytic oxidation of cyclohexane to cyclohexanone/cyclohexanol with oxygen or oxygen-comprising gases by water extraction of the reaction mixture, by esterifying and hydrogenating a substream to 1,6-hexanediol and cyclizing 6-hydroxycaproic ester to ε-caprolactone, which comprises
a) hydrogenating only the aldehydes and ketones present in the aqueous carboxylic acid mixture catalytically to the corresponding alcohols at a hydrogenation temperature of from 50 to 200°C and a reaction pressure of from 5 to 35 bar absolute and degrading more than 50% of the formic acid present in the mixture,
b) reacting the mono- and dicarboxylic acids present in the aqueous reaction mixture, after dewatering, with a low molecular weight alcohol to give the corresponding carboxylic esters,
c) freeing the resulting esterification mixture of excess alcohol and low boilers in a first distillation stage,
d) in a second distillation stage, performing a separation of the bottom product into an ester fraction depleted of 1,4-cyclohexanediols and a fraction comprising 1,4-cyclohexanediols,
e) in a third distillation stage, at least partly removing a stream comprising predominantly 6-hydroxycaproic ester from the ester fraction,
f) catalytically hydrogenating the ester fraction from (e), from which 6-hydroxycaproic ester was removed at least partly, and obtaining 1,6-hexanediol in a manner known per se by distilling the hydrogenation product, and
g) heating the stream comprising predominantly 6-hydroxycaproic ester to temperatures greater than 200°C under reduced pressure, which cyclizes 6-hydroxycaproic ester to ε-caprolactone, and pure ε-caprolactone is obtained from the cyclization product by distillation.

2. The process according to claim 1, wherein the catalyst for the hydrogenation in step a) comprises at least one metal selected from the group consisting of ruthenium, nickel, cobalt, rhenium and copper, in metallic form or as a compound thereof.

3. The process according to claim 1, wherein the catalyst for the hydrogenation in step a) comprises ruthenium, cobalt or nickel supported on shaped titanium dioxide or activated carbon bodies.

4. The process according to claim 3, wherein the catalyst for the hydrogenation in step a) has a metal content in the range from 0.01 to 10% by weight based on the total weight of the catalyst composed of catalytically active metals and support, and a BET surface area in the range from 5 to 500 m²/g measured to DIN 66 131.

5. The process according to any of claims 1 to 4, wherein the catalyst for the hydrogenation in step a) comprises ruthenium.

6. The process according to claim 1, wherein the esterification is performed with alcohols having 1 to 3 carbon atoms.

7. The process according to claim 1, wherein the esterification is performed with alcohols having 4 to 10 carbon atoms.

8. The process according to claim 1, wherein the esterification is performed with methanol and, in the distillation stage (c), a methyl carboxylate fraction essentially free of 1,4-cyclohexanediols is obtained at the top of the column, and a bottom fraction comprising the high boilers and the 1,4-cyclohexanediols, and the methyl carboxylate fraction is transferred into the third distillation stage (d).

9. The process according to claim 1, wherein the esterification is performed with n- or i-butanol and, in the distillation stage (c), the 1,4-cyclohexanediols are removed via the top with the low boilers, and the butyl carboxylates are obtained as a sidestream or as bottoms comprising them and are transferred into the third distillation stage (d).

10. The process according to claim 1, wherein the distillation stages (c) and (d) are performed in a single column.

11. The process according to claim 8, wherein, in the case of esterification with methanol, a fraction comprising essentially methyl dicarboxylates is removed in an upper side draw, an essentially methyl 6-hydroxycaproate fraction as a lower side draw, and a fraction comprising 1,4-cyclohexanediols as the bottom product.

12. The process according to claim 10, wherein, in the case of esterification with n- or i-butanol, a fraction comprising essentially butyl 6-hydroxycaproate is obtained in an upper side draw, a fraction comprising essentially butyl dicarboxylate as a lower side draw, and a fraction comprising the 1,4-cyclohexanediols as the top product.

13. The process according to claim 1, wherein the bottom product of stage (c) is at least partly subjected to a further esterification with further addition of the low molecular weight alcohol and of an esterification catalyst and is separated in a separate distillation stage analogously to (b) and (c), or the further esterification is performed only after removal of the 1,4-cyclohexanediols and the fraction comprising carboxylic esters is introduced into the hydrogenation stage (d).

## Revendications

1. Procédé de préparation de 1,6-hexanediol et d'ε-caprolactone à partir d'un mélange d'acides carboxyliques contenant de l'acide adipique, de l'acide 6-hydroxycaproïque, de l'acide 6-oxocaproïque, de la 4-hydroxycyclohexanone, de l'acide formique et, par rapport à la somme de l'acide adipique et de l'acide hydroxycaproïque, entre 0,5 et 5 % en poids de 1,4-cyclohexanediols, qui est obtenu en tant que produit secondaire de l'oxydation catalytique de cyclohexane en cyclohexanone/cyclohexanol avec de l'oxygène ou des gaz contenant de l'oxygène par extraction aqueuse du mélange réactionnel, par estérification et hydrogénation d'un courant partiel en 1,6-hexanediol et cyclisation d'un ester de l'acide 6-hydroxycaproïque en ε-caprolactone, **caractérisé en ce que**
a) seuls les aldéhydes et cétones contenus dans le mélange aqueux d'acides carboxyliques sont hydrogénés catalytiquement à une température d'hydrogénation de 50 à 200 °C et à une pression de réaction de 5 à 35 bar absolu pour former les alcools correspondants, et plus de 50 % de l'acide formique contenu dans le mélange est décomposé,
b) les acides mono- et dicarboxyliques contenus dans le mélange réactionnel aqueux sont mis en réaction après déshydratation avec un alcool inférieur pour former les esters d'acides carboxyliques correspondants,
c) le mélange d'estérification obtenu est débarrassé de l'alcool en excès et des composés de point d'ébullition bas lors d'une première étape de distillation,
d) une séparation en une fraction ester appauvrie en 1,4-cyclohexanediols et une fraction contenant les 1,4-cyclohexanediols est réalisée à partir du produit de fond lors d'une deuxième étape de distillation,
e) un courant contenant principalement un ester de l'acide 6-hydroxycaproïque est séparé au moins en partie de la fraction ester lors d'une troisième étape de distillation,
f) la fraction ester de (e), de laquelle l'ester de l'acide 6-hydroxycaproïque a été au moins en partie séparé, est hydrogénée catalytiquement et du 1,6-hexanediol est obtenu par distillation du produit d'hydrogénation d'une manière connue en soi, et
g) le courant contenant principalement un ester de l'acide 6-hydroxycaproïque est porté à des températures supérieures à 200 °C à pression réduite, l'ester de l'acide 6-hydroxycaproïque étant cyclisé en ε-caprolactone et de l'ε-caprolactone pure étant obtenue par distillation à partir du produit de cyclisation.

2. Procédé selon la revendication 1, **caractérisé en ce que** le catalyseur pour l'hydrogénation à l'étape a) contient au moins un métal choisi dans le groupe constitué par le ruthénium, le nickel, le cobalt, le rhénium et le cuivre, sous forme métallique ou sous la forme de leur composé.

3. Procédé selon la revendication 1, **caractérisé en ce que** le catalyseur pour l'hydrogénation à l'étape a) contient du ruthénium, du cobalt ou du nickel supporté sur un corps moulé en dioxyde de titane ou en charbon actif.

4. Procédé selon la revendication 3, **caractérisé en ce que** le catalyseur pour l'hydrogénation à l'étape a) présente une teneur en métal dans la plage allant de 0,01 à 10 % en poids, par rapport au poids total du catalyseur constitué de métaux catalytiquement actifs et d'un support, et une surface BET dans la plage allant de 5 à 500 m²/g, mesurée selon DIN 66 131.

5. Procédé selon l'une quelconque des revendications 1 à 4, **caractérisé en ce que** le catalyseur pour l'hydrogénation à l'étape a) contient du ruthénium.

6. Procédé selon la revendication 1, **caractérisé en ce que** l'estérification est réalisée avec des alcools contenant 1 à 3 atomes de carbone.

7. Procédé selon la revendication 1, **caractérisé en ce que** l'estérification est réalisée avec des alcools contenant 4 à 10 atomes de carbone.

8. Procédé selon la revendication 1, **caractérisé en ce que** l'estérification est réalisée avec du méthanol et on obtient à l'étape de distillation (c) une fraction esters méthyliques d'acides carboxyliques essentiellement exempte de 1,4-cyclohexanediols à la tête de la colonne et une fraction de fond contenant les composés de point d'ébullition élevé et les 1,4-cyclohexanediols, et la fraction esters méthyliques d'acides carboxyliques est transférée dans la troisième étape de distillation (d).

9. Procédé selon la revendication 1, **caractérisé en ce que** l'estérification est réalisée avec du n- ou i-butanol et, lors de l'étape de distillation (c), les 1,4-cyclohexanediols sont séparés par la tête avec les composés de point d'ébullition bas et les esters butyliques d'acides carboxyliques sont obtenus en tant que courant latéral ou sous la forme d'un fond les contenant et transférés dans la troisième étape de distillation (d).

10. Procédé selon la revendication 1, **caractérisé en ce que** les étapes de distillation (c) et (d) sont réalisées dans une colonne unique.

11. Procédé selon la revendication 8, **caractérisé en ce qu'**en cas d'estérification avec du méthanol, une fraction contenant principalement des esters méthyliques d'acides dicarboxyliques est séparée dans une sortie latérale supérieure, une fraction principalement d'ester méthylique de l'acide 6-hydroxycaproïque est séparée en tant que sortie latérale inférieure et une fraction contenant les 1,4-cyclohexanediols est séparée en tant que produit de fond.

12. Procédé selon la revendication 10, **caractérisé en ce qu'**en cas d'estérification avec du n- ou i-butanol, une fraction contenant principalement l'ester butylique de l'acide 6-hydroxycaproïque est obtenue dans une sortie latérale supérieure, une fraction contenant principalement des esters butyliques d'acides dicarboxyliques est obtenue en tant que sortie latérale inférieure et une fraction contenant les 1,4-cyclohexanediols est obtenue en tant que produit de tête.

13. Procédé selon la revendication 1, **caractérisé en ce que** le produit de fond de l'étape (c) est soumis au moins en partie à une nouvelle estérification avec ajout supplémentaire de l'alcool inférieur et d'un catalyseur d'estérification, et séparé lors d'une étape de séparation distincte analogue à (b) et (c), ou la nouvelle estérification n'est réalisée qu'après la séparation des 1,4-cyclohexanediols et la fraction contenant des esters d'acides carboxyliques est introduite dans l'étape d'hydrogénation (d).
